# EUROPEAN PATENT APPLICATION

(11) **EP 4 649 913 A1**
(43) Date of publication of application: **19.11.2025**
(21) Application number: 23923749.8
(22) Date of filing: 07.11.2023
(51) Int. Cl.: A61B 34/30, A61B 34/00

(54) **SURGICAL INSTRUMENT AND SURGICAL ROBOT**

(30) Priority: 20.02.2023 CN 202310166356
(71) Applicant: Cornerstone Technology (Shenzhen) Limited, Shenzhen, Guandong 518066 (CN)
(72) Inventor: PAN, Lipeng, Shenzhen, Guangdong 518066 (CN); WANG, Zerui, Shenzhen, Guangdong 518066 (CN)
(74) Representative: Valet Patent Services Limited
(86) International application number: PCT/CN2023/130291
(87) International publication number: WO 2024/174585

(57) **Abstract**

A surgical instrument and a surgical robot are provided. The surgical instrument includes a base assembly, a first pivot assembly, a first flexible transmission assembly, a second pivot assembly, a second flexible transmission assembly, and an execution assembly. The first flexible transmission assembly is configured to drive the first pivot assembly to pivot relative to the base assembly about the first pivot axis. The second flexible transmission assembly is configured to drive the second pivot assembly to pivot relative to the first pivot assembly about the second pivot axis that is not parallel to the first pivot axis. The execution assembly is connected to the second pivot assembly and includes an electrode component and an electric wire for powering the electrode component. The electric wire passes through the second pivot assembly, the first pivot assembly, and the base assembly. The second pivot assembly has an end away from the electrode component and defining a wire passage. The is defined at least by two first wall surfaces facing to each other and a second wall surface transverse to the two first wall surfaces, and the second pivot axis perpendicularly passes through the two first wall surfaces.

## Description

### TECHNICAL FIELD

The present disclosure relates to the field of medical instrument technology, and in particular, to a surgical instrument and a surgical robot including the surgical instrument.

### BACKGROUND

Medical micro-surgical instruments offer advantages such as precise positioning, stable operation, high operational dexterity, extensive working range, and immune to radiation and infection. They are widely used in various surgical procedures. The use of micro-surgical instruments helps improve surgical precision by addressing surgeons' hand tremors, fatigue, and neuromuscular feedback issues. This enables surgeons to operate in optimal comfort, significantly enhancing surgical success rates and reducing patient suffering. In recent years, research in this field has been emerging in medical instrument applications.

### SUMMARY

The summary section introduces a series of simplified conceptual descriptions, which will be further elaborated in the detailed description section. The summary section is not intended to identify key or essential technical features of the claimed technical solution, nor is it intended to be used to determine the scope of the claimed technical solution.

According to a first aspect, the present disclosure provides a surgical instrument including an end effector. The end effector includes a base assembly; a first pivot assembly pivotably connected to the base assembly about a first pivot axis; a first flexible transmission assembly connected to the first pivot assembly, the first flexible transmission assembly being configured to drive the first pivot assembly to pivot relative to the base assembly about the first pivot axis; a second pivot assembly pivotably connected to the first pivot assembly about a second pivot axis that is non-parallel to the first pivot axis; a second flexible transmission assembly connected to the second pivot assembly, the second flexible transmission assembly being configured to drive the second pivot assembly to pivot relative to the first pivot assembly about the second pivot axis; and an execution assembly connected to the second pivot assembly, the execution assembly comprising an electrode component and an electric wire for powering the electrode component, and the electric wire passing through the second pivot assembly, the first pivot assembly, and the base assembly. The second pivot assembly has an end away from the electrode component and defining a wire passage, the electric wire is movably received in the wire passage, the wire passage extends toward the electrode component, the wire passage is defined at least by two first wall surfaces facing each other and a second wall surface transverse to the two first wall surfaces, and the second pivot axis perpendicularly passes through the two first wall surfaces.

According to the present disclosure, the end effector of the surgical instrument can achieve yaw rotation and pitch rotation. The yaw rotation is the rotation of the second pivot assembly, and the pitch rotation is the rotation of the first pivot assembly. The second pivot assembly includes a wire passage for accommodating the electric wire. The axis of the yaw rotation (i.e., the second pivot axis) passes through the side walls of the wire passage, ensuring sufficient space inside the wire passage for the movement of the electric wire. This reduces tension on the electric wire caused by the yaw rotation, which facilitates protecting the electric wire and also contributes to minimizing the size of the second pivot assembly.

According to a second aspect, the present application provides a surgical robot, including a mechanical arm and the surgical instrument according to the first aspect. The surgical instrument is operably connected to the mechanical arm.

### BRIEF DESCRIPTION OF THE DRAWINGS

The following drawings of the present disclosure are presented as a part of the present disclosure for understanding. Embodiments of the present disclosure and description thereof are illustrated in the accompanying drawings to explain the principles of the present disclosure.
FIG. 1 is a perspective schematic diagram of a surgical instrument according to an embodiment of the present disclosure.
FIG. 2 is another perspective schematic diagram of a surgical instrument according to an embodiment of the present disclosure.
FIG. 3 is an exploded view of a surgical instrument according to an embodiment of the present disclosure.
FIG. 4 is another exploded view of a surgical instrument according to an embodiment of the present disclosure.
FIG. 5 is a cross-sectional view of an execution assembly and a second pivot assembly of the surgical instrument in FIG. 1.
FIG. 6 is a perspective schematic diagram of internal structure of a surgical instrument according to an embodiment of the present disclosure.
FIG. 7 is another perspective schematic diagram of internal structure of a surgical instrument according to an embodiment of the present disclosure.
FIG. 8 is another cross-sectional view of an execution assembly and a second pivot assembly of the surgical instrument in FIG. 1.

### DETAILED DESCRIPTION OF THE EMBODIMENTS

In the following description, details are provided to offer a more thorough understanding of the present disclosure. However, it is obvious to those skilled in the art that the present disclosure can be implemented without one or more of these details. In other embodiments, to avoid confusion with the present disclosure, some technical features known in the art are not described.

To fully understand the present disclosure, a detailed description will be provided in the following embodiments. It should be understood that the embodiments are provided to make the disclosure of the present application thorough and complete, and to fully convey the conception of these exemplary embodiments to those skilled in the art. Obviously, the implementation of the embodiments of the present disclosure is not limited to the specific details familiar to those skilled in the art. The preferred embodiments of the present application are described in detail below, but other embodiments may also be included in addition to these detailed descriptions.

The ordinal numbers such as "first" and "second" cited in the present disclosure are merely identifiers and do not have any other meanings, such as specific order, etc. Moreover, the term "first component" itself does not imply the presence of the "second component", and the term "second component" itself does not imply the presence of the "first component". The use of words such as "first", "second", and "third" does not indicate any order and can be interpreted as names.

The terms "distal end" and "proximal end" used in the present disclosure are directional terms commonly used in the field of interventional medical instruments. Here, the "distal end" refers to the end farthest from the operator during a surgical procedure, and the "proximal end" refers to the end closest to the operator during the procedure.

The terms "parallel"/"perpendicular" and similar expressions used in the present disclosure include both absolute parallel/perpendicular relationships and approximate parallel/perpendicular relationships (e.g., relationships within a range of -5° to +5° from absolute parallel/perpendicular), which can achieve the same effect.

The term "length remains unchanged" and similar expressions used in the present disclosure refer to maintaining the original length or fluctuating within a certain range. For example, fluctuations within ±5% of the original length are covered by the term "length remains unchanged" and have an equivalent effect.

The term "rigid material" used in the present disclosure refers to a material has good resistance to deformation, with a small deformation or negligible deformation under external force.

The first aspect of the present disclosure provides a surgical instrument, specifically an electrosurgical instrument. The electrosurgical instrument utilizes highfrequency (radiofrequency) alternating polarity currents applied to biological tissues to perform cutting, coagulation, desiccation, or fulguration procedures. This enables surgical procedures to be completed with minimal bleeding, enhanced surgical efficiency, and improved surgical safety. Typically, the electrosurgical instrument at its distal end has an end effector which is equipped with an electrode tool (such as hook, spatula, forceps, or scissors) which is connected to an electric wire. The electric wire is routed to the proximal end of the electrosurgical instrument to connect to a power supply for transmission of current to the electrode tool. The electrode tool is typically connected to a wrist mechanism. The wrist mechanism is configured to deflect the electrode tool relative to a base assembly. In the embodiments, the wrist mechanism may include a first pivot assembly and a second pivot assembly. The electrosurgical instrument at its proximal end includes a drive mechanism which drives the wrist mechanism through a transmission mechanism to deflect the distal end, thereby accomplishing actions such as cutting, shearing, grasping, clamping, and electrocoagulation.

In minimally invasive surgical applications, miniaturization is one of the leading trends in electrosurgical instrument development. During the design of electrosurgical instrument, the inventors focused on structural compactness to achieve the miniaturization as they had recognized physical constraints on size reduction of each component, beyond which the stiffness and strength of the component would be reduced. The inventors further noted that the wrist mechanism has a significant radial size due to the transmission assembly and the electric wire running through it. In particular, to prevent the electric wire from damage due to stretching during the movement of the wrist mechanism, the current solution involves winding the electric wire several times within the wrist mechanism, which, however, hinders the miniaturization of electrosurgical instrument. Besides, ensuring the stability of the transmission mechanism and the electric wire (e.g., preventing detachment of them) and avoiding their undesired interference are critical challenges that must be addressed for further miniaturization as they are crucial to reliable operation of electrosurgical instrument.

The surgical instrument provided in the embodiments of the present disclosure can address at least one or more of the challenges. Exemplary embodiments according to the present disclosure will be described in detail with reference to the accompanying drawings.

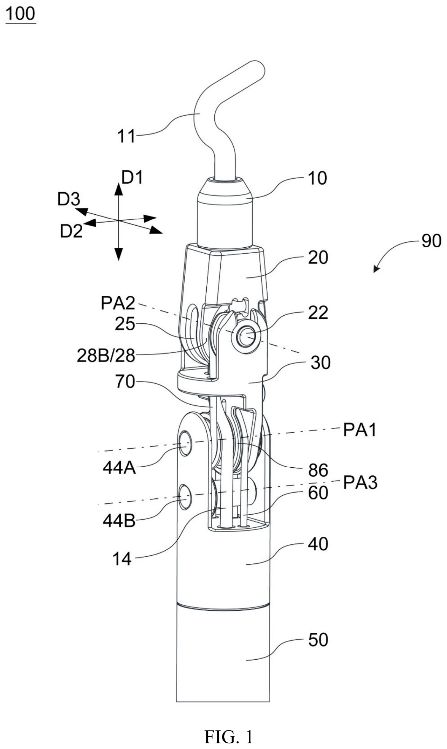

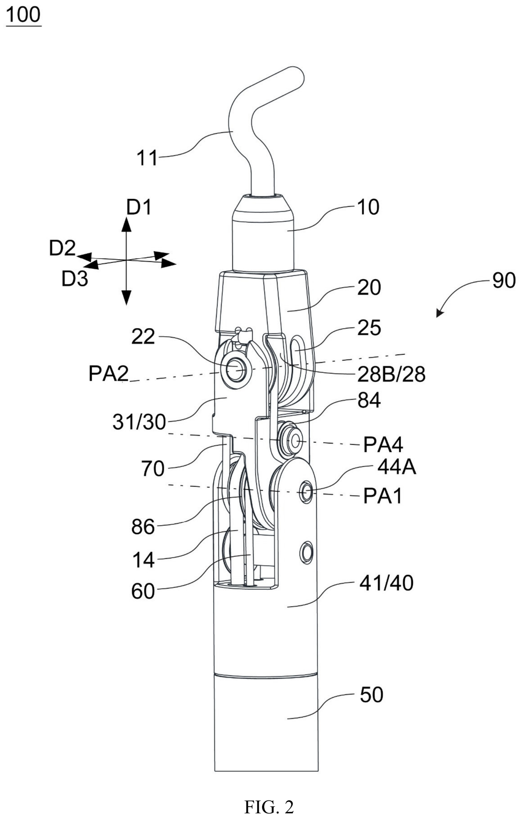

As shown in FIG. 1 and FIG. 2, according to the embodiments of the present disclosure, a surgical instrument 100 includes an end effector 90 at a distal end of the surgical instrument 100. The end effector 90 includes a base assembly 40, an execution assembly 10, a wrist mechanism, and a transmission mechanism. The execution assembly 10 is connected to the base assembly 40 by the wrist mechanism. The wrist mechanism is configured to provide the execution assembly 10 with degrees of freedom relative to the base assembly 40. The transmission assembly is configured to drive the wrist mechanism to move relative to the base assembly 40. The wrist mechanism includes a first pivot assembly 30 and a second pivot assembly 20. The transmission mechanism includes a first flexible transmission assembly 60 and a second flexible transmission assembly 70.

The first pivot assembly 30 is pivotally connected to the base assembly 40 about a first pivot axis PA1. The first pivot axis PA1 extends in a second direction D2. For example, the first pivot assembly 30 may be pivotally connected to the base assembly 40 via a first pivot shaft 44A. The first pivot shaft 44A has the first pivot axis PA1, such that the first pivot assembly 30 is pivotable relative to the base assembly 40 about the first pivot axis PA1. The first flexible transmission assembly 60 is connected to the first pivot assembly 30, and configured to drive the first pivot assembly 30 to pivot relative to the base assembly 40 about the first pivot axis PA1.

The second pivot assembly 20 is pivotally connected to the first pivot assembly 30 about a second pivot axis PA2. The second pivot axis PA2 extends in a third direction D3. For example, the second pivot assembly 20 may be pivotally connected to the first pivot assembly 30 via a second pivot shaft 22. The second pivot shaft 22 has the second pivot axis PA2, such that the second pivot assembly 20 is pivotable relative to the first pivot assembly 30 about the second pivot axis PA2. The second flexible transmission assembly 70 is connected to the second pivot assembly 20, and configured to drive the pivot assembly 20 to pivot relative to the first pivot assembly 30 about the second pivot axis PA2. The second pivot axis PA2 is not parallel to the first pivot axis PA1. In some embodiments, the second pivot axis PA2 is perpendicular to the first pivot axis PA1. As a result, the execution assembly 10 (i.e., an electrode component 11 described below) deflects in two mutually perpendicular directions, achieving movement in two degrees of freedom.

The execution assembly 10 is mounted to the second pivot assembly 20. The execution assembly 10 includes an electrode component 11 and an electric wire 14 for powering the electrode component 11. It can be understood that the electric wire 14 has an end connected to the electrode component 11. The electric wire 14 passes through the second pivot assembly 20, the first pivot assembly 30, and the base assembly 40. In the illustrated embodiments, the electrode component 11 is configured as an electrically conductive hook. It can be understood that, in other embodiments, the electrode component 11 may be configured as an electrically conductive spatula, forceps, or scissors, which is not limited herein.

In the embodiments, the rotation of the second pivot assembly 20 about the second pivot axis PA2 results in yaw of the end effector 90 (or the electrode component 11), and the rotation of the first pivot assembly 30 about the first pivot axis PA1 results in pitch of the end effector 90 (or the electrode component 11). Thus, the first pivot assembly 30 and the second pivot assembly 20 provide the execution component 10 with two degrees of freedom for executing relevant operations during surgery.

In some embodiments, the maximum rotation angle of the second pivot assembly 20 may be approximately ±90° (i.e., the yaw angle is about ±90 °), and the maximum rotation angle of the first pivot assembly 30 may also be approximately ±90° (i.e., the pitch angle is about ±90°).

It can be understood that the surgical instrument 100 has a proximal end connected to a driving device. The driving device is configured to control the yaw and pitch of the end effector 90 through the transmission mechanism. The driving device may be an electric motor or a manually operatable handle for the surgeon. The proximal end of the surgical instrument 100 is further connected to an energy generator that is configured to supply required current to the electrode component 11. The electric wire 14 is routed to the proximal end of the surgical instrument 100 and electrically connected to the energy generator.

In some embodiments, the second pivot assembly 20 has an end away from the electrode component 11 and defining a wire passage 25 for accommodating the electric wire 14. That is, the wire passage 25 has an opening defined at the proximal end of the second pivot assembly 20. In the present disclosure, the distal end refers to the end of the surgical instrument 100 or its component away from the operator, while the proximal end refers to the end of the surgical instrument 100 or its component close to the operator. The electric wire 14 is movably received within the wire passage 25. The wire passage 25 extends toward the electrode component 11, allowing the electric wire 14 to connect to the electrode component 11. In other words, the opening of the wire passage 25 is adjacent to the first pivot assembly 30, and the bottom of the wire passage 25 is adjacent to the execution component 10.

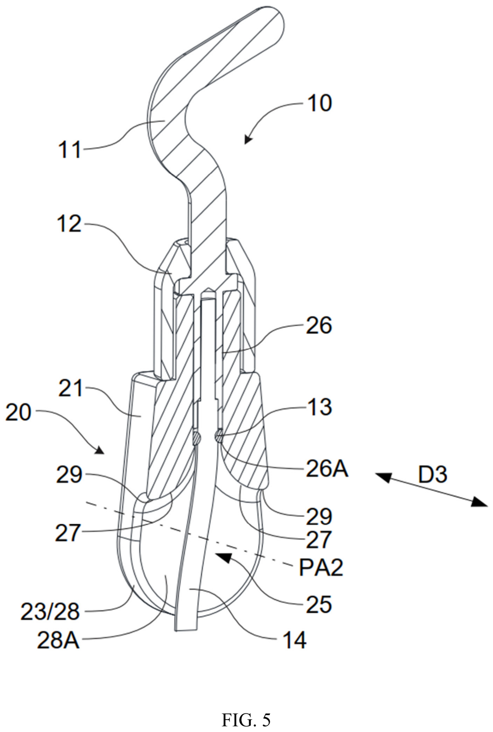

Further, referring to FIG. 5, the wire passage 25 is defined at least by two first wall surfaces 28A perpendicular to the third direction D3 (i.e., the second pivot axis PA2) and a second wall surface 29 transverse to the first wall surfaces 28A. The two first wall surfaces 28A are spaced apart and face each other. The second wall surface 29 transversing to the first wall surfaces 28A means that the second wall surface 29 does not extend parallel to the first wall surfaces 28A but instead intersects with the first wall surfaces 28A, either intersecting perpendicularly or at other angles. In the embodiments, the first wall surfaces 28A may be understood as the side wall surfaces of the wire passage 25, and the second wall surface 29 may be understood as the bottom wall surface of the wire passage 25. The second pivot axis PA2 passes through the two first wall surfaces 28A. That is, the wire passage 25 extends beyond the second pivot axis PA2 in the direction toward the electrode component 11, which provides sufficient space for movements of the electric wire 14, allowing it to be retracted or released during pivoting of the second pivot assembly 20 about the second pivot axis PA2.

During yaw of the second pivot assembly 20, with the configuration of the wire passage 25, the electric wire 14 is prevented from over stretching or undesired interference with the second pivot assembly 20, without the need for being wound in multiple coils within the wrist mechanism. Furthermore, by routing the electric wire 14 through the wire passage 25, on one hand, it reduces the space occupied by the electric wire 14 within the wrist mechanism, minimizing the radial dimension of the wrist mechanism and thus contributing to the miniaturization of the electrosurgical instrument; on the other hand, it prevents undesired interference (e.g., entanglement) between the electric wire 14 and the transmission mechanism during the movement of the second pivot assembly 20.

In some embodiments, the two first wall surfaces 28A are parallel with each other and extend flatly in a direction perpendicular to the third direction D3. The distance between the two first wall surfaces 28A (i.e., the dimension of the wire passage 25 in the third direction D3) is matched to the diameter of the electric wire 14. Herein, the wording "matched" means that the width of the wire passage 25 is slightly greater than the diameter of the electric wire 14. In some embodiments, the width of the wire passage 25 is 1.1 to 1.5 times the diameter of the electric wire 14. As such, during yaw of the second pivot assembly 20, the electric wire 14 barely undergoes displacement in the third direction D3, but primarily deflects within a plane perpendicular to the third direction D3. That is, the two first wall surfaces 28A restrict the movement of the electric wire 14 in the third direction D3, while also helping to reduce the dimension of the second pivot assembly 20 in the third direction D3.

To minimize undesired interference between the electric wire 14 and the second pivoting assembly 20 during pivoting, for example, in a case of a maximum pivoting angle of the second pivot assembly 20 being approximately ±90°, the second wall surface 29 and the execution assembly 10 (i.e., the electrode component 11) are located on the same side of the second pivot axis PA2, i.e., the second wall surface 29 is located between the second pivot axis PA2 and the execution assembly 10. This ensures sufficient space for movements of the electric wire 14, preventing or reducing stretching of the electric wire 14 when the second pivot assembly 20 reaches its extreme positions, thereby protecting the electric wire 14 and also enabling smoother rotation of the second pivot assembly 20.

In some embodiments, the second wall surface 29 may be configured as a flat surface, a curved surface, or a combination of a flat surface and a curved surface. In the embodiments, the second wall surface 29 includes a third curved surface 27 featuring a third arc parallel to the first wall surface 28A. That is, the axis of the third curved surface 27 is parallel to the second pivot axis PA2. The third curved surface 27 is configured to limit the movement range of the electric wire 14 during rotation of the second pivot assembly 20 relative to the first pivot assembly 30, to prevent excessive bending of the electric wire 14. In the embodiments, the second pivot assembly 20 is configured with symmetrical maximum pivoting angles, and accordingly the third curved surface 27 is configured as a symmetrical structure.

In some embodiments, the second wall surface 29 may be recessed toward the execution assembly 10 to define a wire groove (not labeled) contoured to match the shape of the electric wire 14. This is, the shape of the second wall surface 29 matches the that of the electric wire 14, thereby providing improved guidance for the electric wire 14.

In some embodiments, to facilitate the stability of the connection between the electric wire 14 and the electrode component 11, the second pivot assembly 20 has a distal end (the end adjacent to the electrode component 11) defining a wire through-hole 26 for accommodating the electric wire 14. The wire through-hole 26 is communicated with the wire passage 25. The wire through-hole 26 has an opening located at its end and defined in the second wall surface 29 of the wire passage 25. The electric wire 14 passes through the wire passage 25 and enters the wire through-hole 26. The electric wire 14 may be connected to the electrode component 11 inside the wire through-hole 26, or the electric wire 14 may extend out of the wire through-hole 26 and be connected to the electrode component 11. The connection may be implemented by means of, for example, crimping or welding, ensuring low impedance and preventing overheating at their junction. The inner diameter of the wire through-hole 26 is approximately equal to the diameter of the portion of the electric wire 14 inserted into the wire through-hole 26 or the diameter of the portion of the electrode component 11 inserted into the wire through-hole 26.

In the embodiments, the wire through-hole 26 has an inner wall connected to the third curved surface 27 of the second wall surface 29. Or in other words, the second wall surface 29 transitions to the inner wall of the wire through-hole 26 via the third curved surface 27. In some embodiments, the third curved surface 27 is tangent to the inner wall of the wire through-hole 26, allowing the third curved surface 27 to smoothly transition to the inner wall of the wire through-hole 26. This ensures that the electric wire 14 will not be excessively bent at the transition area during rotation of the second pivot assembly 20, thereby better protecting the electric wire 14. In some embodiments, the third curved surface 27 has an end extending away from the wire through-hole 26 to the opening of the wire passage 25, thereby guiding the electric wire 14 throughout the yaw of the second pivot assembly 20.

In some embodiments, as shown in FIG. 1 to FIG. 4, the second pivot assembly 20 includes a second base 21. The second base 21 has a proximal end pivotally connected to the first pivot assembly 30 via the second pivot shaft 22, and a distal end connected to the execution assembly 10. It can be understood that the second base 21 is configured as a hollow structure for accommodating the electric wire 14. In some embodiments, referring to FIG. 5, the hollow structure includes the wire passage 25 and the wire through-hole 26. The wire passage 25 is defined at the proximal end of the second base 21, the wire through-hole 26 is defined at the distal end of the second base 21, and the wire passage 25 is communicated with the wire through-hole 26. The electric wire 14 is inserted into the wire through-hole 26 and connected to the electrode component 11.

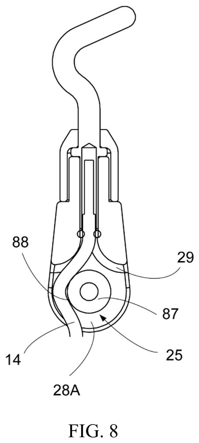

In some embodiments, referring to FIG. 8, the wire passage 25 may have a guiding surface 88 configured to guide the electric wire 14. Apart from the guiding surface 88, the structures illustrated in FIG. 8 are similar to those in FIG. 5 and may refer to the description of FIG. 5, which is not detailed herein. When the second pivot assembly 20 is in the neutral position relative to the first pivot assembly 30, the electric wire 14 rides on the guiding surface 88. The guiding surface 88 may be configured as a cylindrical surface extending around the second pivot axis PA2. With reference to FIG. 8, when the second pivot assembly 20 rotates clockwise relative to the first pivot assembly 30, the electric wire 14 rides more on the guiding surface 88. Conversely, when the second pivot assembly 20 rotates counterclockwise relative to the first pivot assembly 30, the electric wire 14 partially separates from the guiding surface 88 but is prevented by the guiding surface 88 from extending out through the opening of the wire passage 25. Thus, the configuration of the guiding surface 88 makes the electric wire 14 to be confined within the wire passage 25 throughout the rotation of the second pivot assembly 20 relative to the first pivot assembly 30. Further, the guiding surface 88 may be configured to rotate about the second pivot axis PA2 to reduce or avoid sliding friction between the electric wire 14 and the guiding surface 88. In some embodiments, a guiding pulley 87 may be provided in the wire passage 25. The guiding pulley 87 is rotatably mounted relative to the second base 21 about the second pivot axis PA2, and the electric wire 14 rides on the guiding pulley 87. That is, the outer peripheral surface of the guiding pulley 87 serves as the guiding surface 88 or part of the guiding surface 88.

The second base 21 includes two third side surfaces 28B that are transverse to the second pivot axis PA2 and spaced apart in the direction of the second pivot axis PA2. The second pivot shaft 22 may be arranged on the two third side surfaces 28B. The second flexible transmission assembly 70 is connected to the second base 21, to drive the second base 21 to rotate relative to the first pivot assembly 30. The second flexible transmission assembly 70 is located outside the wire passage 25, thereby avoiding undesired interference with the movement of the electric wire 14 within the wire passage 25.

To facilitate the rotation of the second pivot assembly 20 relative to the first pivot assembly 30, the second base 21 has a second curved surface 23 at its proximal end. The second curved surface 23 is axially centered about the second pivot axis PA2. The second curved surface 23 features a second arc parallel to the first wall surface 28A, and the central angle of the second arc is related to the maximum rotation angle of the second pivot assembly 20. The opening of the wire passage 25 extends along the second arc at least to both ends of the second arc, ensuring that during rotation of the second pivot assembly 20, neither end of the opening of the wire passage 25 will exert pulling force on the electric wire 14. In the embodiments, since the maximum rotation angle of the second pivot assembly 20 is approximately ±90°, the central angle of the second arc is greater than or equal to 180 degrees. In the embodiments, the opening of the wire passage 25 extends along the second arc and beyond both ends of the second arc. This is because the electric wire 14 has a physical outer contour, and the extended length beyond each end is greater than or equal to the radius of the electric wire 14. This design prevents local bending of the electric wire 14 when the second pivot assembly 20 reaches its maximum rotation angle.

The first pivot assembly 30 includes a first base 31. The first base 31 includes a base plate 35, a connecting arm 37, and a support arm 38. The base plate 35 extends in a direction parallel to both the second pivot axis PA2 and the first pivot axis PA1. The base plate 35 defines one or more through-holes through which the electric wire 14 and the second flexible transmission assembly 70 pass. The connecting arm 37 is located on one side of the base plate 35, and the support arm 38 is located on the other side of the base plate 35. Both the connecting arm 37 and the support arm 38 are connected to the base plate 35.

The connecting arm 37 is connected to the side of the base plate 35 facing the base assembly 40 and configured to be pivotably connected to the base assembly 40. The connecting arm 37 is rotatably fitted onto a first pivot shaft 44A, such that the first pivot assembly 30 rotatable about the first pivot shaft 44A. The connecting arm 37 is configured as a plate-like structure. In some embodiments, the connecting arm 37 extends perpendicularly to the base plate 35. The first flexible transmission assembly 60 is connected to the connecting arm 37 to drive the rotation of the first base 31 relative to the base assembly 40.

The support arm 38 is connected to the side of the base plate 35 facing the second pivot assembly 20, and configured to support and connect the second pivot assembly 20. In the embodiments, two support arms 38 faced with each other are provided. In some embodiments, the two support arms 38 extend perpendicularly to the base plate 35. In some embodiments, the extension plane of the connecting arm 37 is perpendicular to the extension planes of the two support arms 38. The space between the two support arms 38 is configured to accommodate the second pivot assembly 20.

The second pivot shaft 22 has two ends, each of which is connected to a respective one of the two support arms 38. Consequently, the two support arms 38 are spaced apart in the extension direction of the second pivot axis PA2. In some embodiments, each of the two support arms 38 defines a notch 39, and the two notches 39, respectively on the two support arms 38, are aligned along the second pivot axis PA2. The two ends of the second pivot shaft 22 are seated in the two notches 39 and secured by end caps 32 (which restrict the second pivot shaft 22 within the two notches 39), thereby stably connecting the second pivot shaft 22 to the first pivot assembly 30.

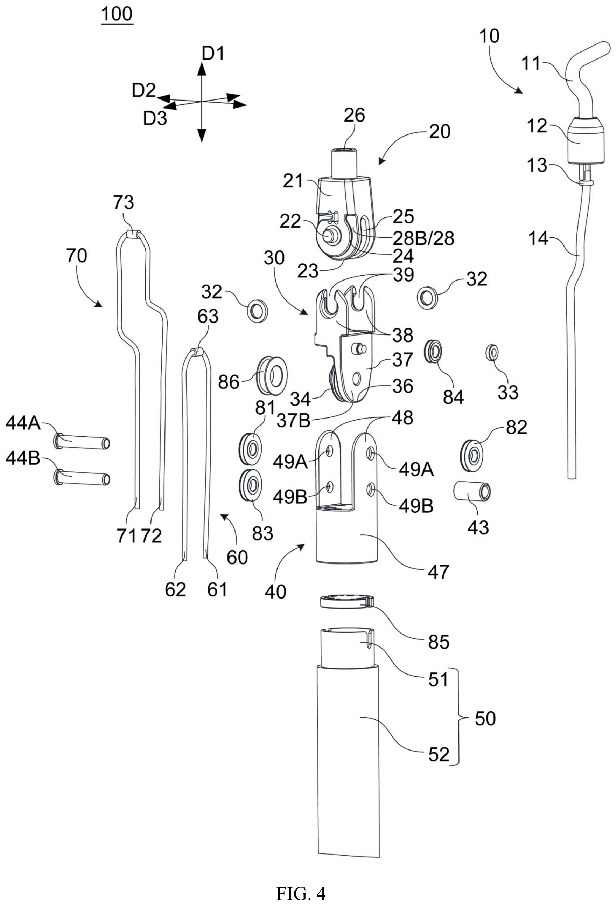

In some embodiments, the connecting arm 37 extends in a direction perpendicular to the second direction D2. The base plate 35 has an end in the second direction D2, which is connected to the connecting arm 37. The connecting arm 37 forms a side wall of the first base 31. It can be understood that the connecting arm 37 has an end connected to the base plate 35 and another end away from the base plate 35. In some embodiments, to facilitate the rotation of the first pivot assembly 30 relative to the base assembly 40, the end of the connecting arm 37 away from the base plate 35 has a surface configured as a first curved surface (referring to FIG. 4), and the first curved surface has an arc centered on the first pivot axis PA1.

The base assembly 40 includes a support base 41 and the first pivot shaft 44A. The support base 41 extends in the first direction D1, which is also referred to as a longitudinal axis direction of the base assembly 40. As shown in the drawings, the first direction D1 is the up-down direction. In some embodiments, the first direction D1 is perpendicular to the first pivot axis PA1. The support base 41 includes a support body 47 and two support upright arms 48 arranged in the first direction D1, and the support body 47 and the two support upright arms 48 extend away from each other. The support body 47 is configured as a cylindrical structure with its axial direction being the first direction D1. The two support upright arms 48 extend from the support body 47 in the first direction D1 and arranged to face each other. The first pivot shaft 44A has two ends, each of which is connected to a respective one of the two support upright arms 48, such that the two support upright arms 48 are spaced apart in the extension direction of the first pivot axis PA1 (i.e., the second direction D2). The space between the two support upright arms 48 is configured to accommodate at least the first pivot assembly 30 and the electric wire 14.

In some embodiments, each of the two support upright arms 48 defines a first through-hole 49A. The two first through-holes 49A are aligned along the first pivot axis PA1. The two ends of the first pivot shaft 44A are seated in the two first through-holes 49A, respectively, thereby securely mounting the first pivot shaft 44A to the support base 41 of the base assembly 40. The two support upright arms 48 are configured to support and connect the first pivot assembly 30.

The support base 41 further includes a base bottom plate 46, which is located at the connection between the support body 47 and the two support upright arms 48. The base bottom plate 46 serves as the top cover plate of the support body 47. The support body 47 is located on one side of the base bottom plate 46, and two support upright arms 48 are located on the other side of the base bottom plate 46. The support body 47 and the two support upright arms 48 are all connected to the base bottom plate 46. In some embodiments, the base bottom plate 46 extends in a direction perpendicular to the first direction D1. As shown in the drawings, the base bottom plate 46 is configured as a horizontal plate. The base bottom plate 46 defines one or more through-holes through which the electric wire 14 and the transmission mechanism pass.

To guide the electric wire 14 between the two support upright arms 48, the end effector 90 is further provided with a wire pulley 86. The wire pulley 86 is rotatably connected to the base assembly 40 about the first pivot axis PA1. In some embodiments, the wire pulley 86 is rotatably fitted onto the first pivot shaft 44A. The wire pulley 86 defines a groove along which the electric wire 14 rides, such that the electric wire 14 is positioned and guided by the wire pulley 86.

The wire passage 25 constrains movement of the electric wire 14 in the third direction D3 at a location nearer the distal end of the electric wire 14. The cable pulley 86 constrains movement of the electric wire 14 in the second direction D2 at a location nearer the proximal end of the electric wire 14. By collaboratively positioning the electric wire 14 using both the cable pulley 86 and the wire passage 25, the electric wire 14 maintains a smooth extension. This prevents undesired interference between the electric wire 14 and the transmission mechanism during movement of the wrist mechanism.

When the first pivot assembly 30 is in the neutral position relative to the base assembly 40, the first direction D1 is perpendicular to the second pivot axis PA2. In the illustrated embodiments, when the first pivot assembly 30 and the second pivot assembly 20 are both in the neutral positions, the surgical instrument 100 exhibits an elongated structure extending in the first direction D1, with the base assembly 40, the first pivot assembly 30, the second pivot assembly 20, and the execution assembly 10 arranged sequentially from the proximal end to the distal end substantially in the first direction D1. Here, the first direction D1 may be understood as the longitudinal axis direction of the end effector 90.

In some embodiments, the second flexible transmission assembly 70 is configured as a drive cable (e.g., made from steel, or tungsten, etc.). The drive cable is connected to a second fastener 73, and is further connected to the second base 21 via the second fastener 73. When the lengths of the drive cable on both sides of the second fastener 73 change, the second flexible transmission assembly 70 rotates about the second pivot axis PA2, realizing the yaw of the end effector 90. The second fastener 73 is secured to the second base 21, allowing for synchronous movement between the second base 21 and the second fastener 73. The use of the drive cable to achieve the rotation (deflection) of the second pivot assembly 20 is conducive to reducing the size of the surgical instrument 100.

In some embodiments, the second flexible transmission assembly 70 includes a first cable 71 and a second cable 72. In an assembled state of the surgical instrument 100, the first cable 71 and the second cable 72 are positioned radially opposed about the second pivot shaft 22.

In some embodiments, the extension trajectory of the first cable 71 in the second pivot assembly 20 and that of the second cable 72 in the second pivot assembly 20 lie in a same plane (i.e., a first plane). The first plane is perpendicular to the second pivot axis PA2 and located outside the wire passage 25. The second flexible transmission assembly 70 being arranged outside the wire passage 25 may prevent undesired interference with the electric wire 14. In some embodiments, the first cable 71 and the second cable 72 may be two segments of a single drive cable. As shown in the figures, the second fastener 73 is arranged at the middle of the single drive cable, dividing the single drive cable into the first cable 71 and the second cable 72 positioned opposed about the second fastener 73. In other embodiments (not shown), the first cable 71 and the second cable 72 are two unconnected drive cables. That is, the first cable 71 and the second cable 72 are two independent drive cables, each having its own fastener that is fixed to the second base 21.

In some embodiments, the extension trajectory of the first cable 71 within the second pivot assembly 20 lies in a first plane, and the extension trajectory of the second cable 72 within the second pivot assembly 20 lies in a second plane. The first plane and the second plane are parallel to each other and perpendicular to the second pivot axis PA2. The wire passage 25 is located between the first plane and the second plane. In this configuration, the first cable 71 and the second cable 72 are two unconnected cables. That is, the first cable 71 and the second cable 72 are two independent drive cables, each having its own fastener that is fixed to the second base 21.

In some embodiments, the first plane and the second plane are coplanar with the two third side surfaces 28B of the second base 21, respectively. In other words, the first cable 71 and the second cable 72 are both connected to a common one of the two third side surfaces 28B; or the first cable 71 is connected to one of the two third side surfaces 28B, and the second cable 72 is connected to the other one of the two third side surfaces 28B.

In some embodiments, as shown in FIG. 1 to FIG. 4, the third side surface 28B of the second base 21 defines a second cable groove 24 for guiding the first cable 71 and the second cable 72. In some embodiments, the second cable groove 24 is configured as an arc-shaped groove (with a central angle of at least 180 degrees) centered about the second rotation axis PA2. In a radial direction of the second cable groove 24, the first cable 71 and the second cable 72 extend along opposite sides of the second cable groove 24, both connecting to the same third side surface 28B. The second fastener 73 is arranged on the outer surface of the second base 21 corresponding to the second curved surface 23, facilitating routing of the first cable 71 and the second cable 72 along radially opposed sides of the second pivot shaft 22.

In other embodiments, each of the two third side surfaces 28B defines a cable groove (not shown) for guiding one of the first cable 71 and the second cable 72. In this configuration, the first cable 71 is connected to one third side surface 28B and the second cable 72 is connected to the other third side surface 28B.

In yet other embodiments, the first plane and the second plane may not be coplanar with the two third side surfaces 28B of the second base 21, but instead positioned closer to the wire passage 25.

The first cable 71 and the second cable 72 extend from the second pivot assembly 20 to the base assembly 40, spanning a significant length. To maintain stable positioning of the first cable 71 and the second cable 72, the present disclosure further constrains both the first cable 71 and the second cable 72 via a pulley system.

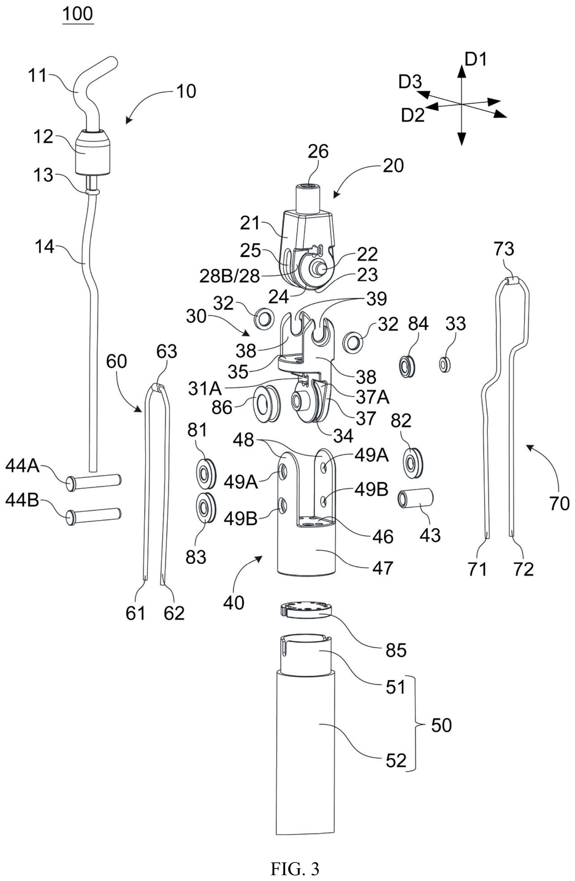

As shown in FIG. 3, FIG. 4, FIG. 6, and FIG. 7, the surgical instrument 100 further includes a first pulley 81 configured to guide the first cable 71 and a second pulley 82 configured to guide the second cable 72. Both the first pulley 81 and the second pulley 82 are pivotally connected to the base assembly 40 about the first pivot axis PA1. In some embodiments, the first pulley 81 and the second pulley 82 are both rotatably fitted onto the first pivot shaft 44A and spaced apart along the first pivot axis PA1. The first cable 71 rides on the first pulley 81, and the second cable 72 rides on the second pulley 82. As a result, the first pulley 81 constrains and guides the first cable 71, and the second pulley 82 constrains and guides the second cable 72. In some embodiments, the first cable 71 rides on the first pulley 81 in the opposite direction to the second cable 72 on the second pulley 82. This prevents the first pivot assembly 30 from experiencing kinematic lock due to the first cable 71 and the second cable 72 in the pitch of the end effector 90. In some embodiments, the wire pulley 86 is located between the first pulley 81 and the second pulley 82. In some embodiments, the wire pulley 86 is located on one side of the connecting arm 37, and the second pulley 82 is located on the other side of the connecting arm 37. That is, the wire pulley 86 is located between the first pulley 81 and the connecting arm 37.

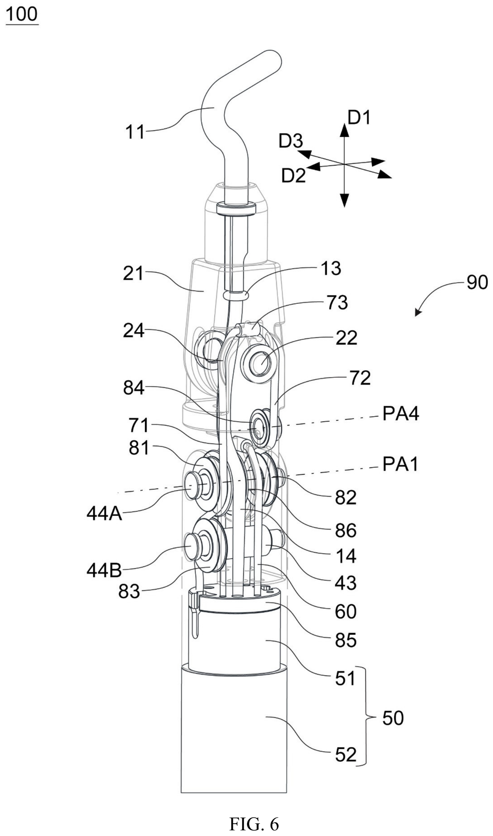

To better position the first cable 71, the surgical instrument 100 further includes a third pulley 83 pivotally connected to the base assembly 40 about a third pivot axis PA3. The base assembly 40 is provided with a third pivot shaft 44B. In some embodiments, each of the two support upright arms 48 defines a second through-hole 49B, and two second through-holes 49B are aligned in the first direction D1. The third pivot shaft 44B has two ends seated in the two second through-holes 49B, thereby stably securing the third pivot shaft 44B to the base assembly 40. As shown in FIG. 1 and FIG. 2, the third pivot shaft 44B has the third pivot axis PA3, which is parallel to the first pivot axis PA1. The third pulley 83 is rotatably fitted onto the third pivot shaft 44B. The first pivot axis PA1 and the third pivot axis PA3 define a third plane. As shown in FIG. 6, the first cable 71 rides on the first pulley 81 on one side of the third plane and on the third pulley 83 on the opposite side of the third plane. The first cable 71 is positioned on both sides of the third plane by the first pulley 81 and third pulley 83, preventing derailment from the two pulleys during pitch of the end effector 90, thereby enhancing stability of the first cable 71.

Further, given the considerable span where the first cable 71 extends from the second cable groove 24 of the second base 21 to the first pulley 81, the base plate 35 on the first base 31 provides supplementary constraint along this path. In some embodiments, the base plate 35 may define a through-hole through which the first cable 71 passes. Constrained by the base plate 35, the first cable 71 maintains alignment with the cable groove of the first pulley 81 throughout the pitch of the end effector 90.

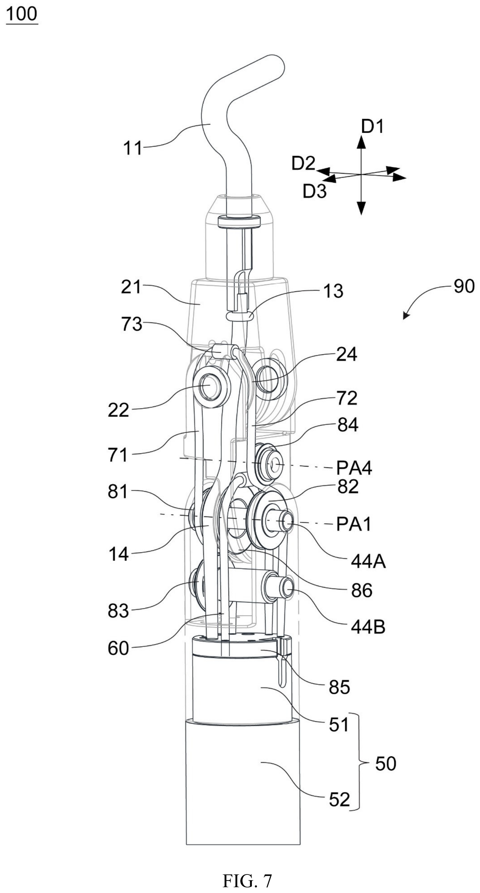

To redirect the second cable 72 such that the first cable 71 rides on the first pulley 81 in the opposite direction to the second cable 72 on the second pulley 82, and also to better position the second cable 72, the surgical instrument 100 further includes a fourth pulley 84 connected to the first pivot assembly 30. The fourth pulley 84 has an axis PA4 fixed relative to the first pivot assembly 30, that is, the fourth pulley 84 is movable along with the first pivot assembly 30. Additionally, the fourth pulley 84 is rotatable relative to the first pivot assembly 30 about the axis PA4. The axis PA4 of the fourth pulley 84 is parallel to the first pivot axis PA1, such that the first pivot axis PA1 and the axis PA4 of the fourth pulley 84 define a fourth plane. As shown in FIG. 7, the second cable 72 rides on the fourth pulley 84 on one side of the fourth plane and on the second pulley 82 on the opposite side of the fourth plane. The second cable 72 is positioned on both sides of the fourth plane by the second pulley 82 or the fourth pulley 84, preventing derailment from the two pulleys during pitch of the end effector 90, thereby enhancing stability of the second cable 72.

In some embodiments, the fourth pulley 84 is disposed on the connecting arm 37, and a pulley cap 33 is connected to the connecting arm 37 to limit the axial position of the fourth pulley 84.

In some embodiments, the connecting arm 37 includes a first side surface 37A and a second side surface 37B facing away from the first side surface 37A, and the two side surfaces are transverse to (e.g., perpendicular to) the first pivot axis. The first flexible transmission assembly 60 is connected to the first side surface 37A of the connecting arm 37. The first pulley 81 and the wire pulley 86 are located on the same side of the connecting arm 37, i.e., the side of the first side surface 37A. The fourth pulley 84 and the second pulley 82 are located on the same side of the connecting arm 37, i.e., the second side surface 37B. The connecting arm 37 physically separates the first cable 71 and the second cable 72, preventing undesired interference between the first cable 71 and the second cable 72.

In the illustrated embodiments, the path of the first cable 71 is guided by the second cable groove 24, the first pulley 81, the third pulley 83, and the through-hole in the base bottom plate 46. In some embodiments, when the first pivot assembly 30 is in the neutral position relative to the base assembly 40, the first cable 71 extends from the second cable groove 24 of the second pivot assembly 20 in the first direction D1 to the first pulley 81. It bends at the first pulley 81 to penetrate the third plane, and rides on the third pulley 83. It further extends in the first direction D1 to the through-hole in the base bottom plate 46 of the base assembly 40. This confines the section of the first cable 71 spanning from the exit point of the second cable groove 24 (where it leaves the second cable groove 24) to the through-hole in the base bottom plate 46 of the base assembly 40 within a fifth plane parallel to both the first direction D1 and the third direction D3 during pitch and yaw of the end effector 90. Consequently, the first cable 71 can extend smoothly without interference (e.g., free from undesired interference with the walls of the second cable groove 24 and/or the pulley groove walls of the first pulley 81), contributing to stable force distribution within the first cable 71.

Understandably, to achieve the aforementioned effect, the dimensions and positions of the second cable groove 24, the first pulley 81, and the third pulley 83 need to be matched. For example, the groove extension trajectory of the first pulley 81 lies in the same plane as that of the third pulley 83, and this plane is tangent to the arc of the second cable groove 24. This allows the first cable 71 to extend maximally within the same plane, thereby facilitating smooth operation.

In the illustrated embodiments, the path of the second cable 72 is guided by the second cable groove 24, the fourth pulley 84, the second pulley 82, and the through-hole in the base bottom plate 46. In some embodiments, when the first pivot assembly 30 is in the neutral position relative to the base assembly 40, the second cable 72 extends from the second cable groove 24 of the second pivot assembly 20 in the first direction D1 to the fourth pulley 84. It bends at the fourth pulley 84 to penetrate the fourth plane, and rides on the second pulley 82. It further extends in the first direction D1 to the through-hole in the base bottom plate 46 of the base assembly 40. This ensures confines the section of the second cable 72 spanning from the exit point of the second cable groove 24 (where it leaves the second cable groove 24) to the through-hole in the base bottom plate 46 of the base assembly 40 within a sixth plane that is parallel to both the first direction D1 and the third direction D3 during pitch and yaw of the end effector 90. Consequently, the second cable 72 can extend smoothly without interference (e.g., free from undesired interference with the walls of the second cable groove 24 and/or the pulley groove walls of the fourth pulley 84), contributing to stable force distribution within the second cable section 72. Furthermore, the fifth plane and the sixth plane are parallel and spaced apart, preventing undesired interference between the first cable 71 and the second cable 72.

Understandably, to achieve the aforementioned effect, the dimensions and positions of the second cable groove 24, the fourth pulley 84, and the second pulley 82 need to be matched. For example, the groove extension trajectory of the fourth pulley 84 lies in the same plane as that of the second pulley 82, and this plane is tangent to the arc of the second cable groove 24. This allows the second cable 72 to extend maximally within the same plane, thereby facilitating smooth operation.

In some embodiments, the first pivot shaft 44A and the third pivot shaft 44B are arranged in the first direction D1. The first pivot shaft 44A may be located above the third pivot shaft 44B or below the third pivot shaft 44B. In some embodiments, the first pivot shaft 44A and the third pivot shaft 44B may have the same structure. In some embodiments, the first pulley 81, the second pulley 82, and the third pulley 83 may be configured as identical pulleys.

Understandably, the electric wire 14 is positioned by the wire pulley 86 on the first pivot shaft 44A, and thus passes by one side of the third pivot shaft 44B. In some embodiments, to prevent friction between the electric wire 14 and the third pivot shaft 44B (e.g., made from metallic material) from damaging the insulation layer of the electric wire 14, a shaft sleeve 43 is fitted over the third pivot shaft 44B to contact the electric wire 14. The shaft sleeve 43 is rotatable about the third pivot shaft 44B, thereby minimizing friction with the electric wire 14. Moreover, the sleeve 43 restricts movement of the third pulley 83 in the second direction D2 (i.e., along the third pivot axis PA3).

In some embodiments, similar to the second flexible drive assembly 70, the first flexible drive assembly 60 is configured as a drive cable. The first flexible drive assembly 60 includes a third cable 61 and a fourth cable 62. In some embodiments, the third cable 61 and the fourth cable 62 may be segments of a single drive cable, and a first fastener 63 is arranged at the middle of the single drive cable, dividing the single drive cable into the third cable 61 and the fourth cable 62. The first fastener 63 is arranged at the connecting part 31A of the first base 31 (as shown in FIG. 3). In some embodiments, the first fastener 63 may be secured to the connecting part 31A, allowing for synchronous movement between the first base 31 and the first fastener 63. When the lengths of the third cable 61 and the fourth cable 62 change, the first pivot assembly 30 rotates, realizing the pitch of the end effector 90. In other embodiments, the third cable 61 and the fourth cable 62 are two unconnected drive cables. That is, the third cable 61 and the fourth cable 62 are two independent drive cables, each having its own fastener that is fixed to the first base 31.

In some embodiments, the connecting part 31A is arranged on the connecting arm 37. In some embodiments, when the first pivot assembly 30 is in the neutral position, the connecting part 31A and the first pivot shaft 44A are aligned in the first direction D1. The third cable 61 and the fourth cable 62 are located radially opposed about the first pivot shaft 44A.

The connecting arm 37 defines a first cable groove 34 for guiding the third cable 61 and the fourth cable 62. In some embodiments, the first cable groove 34 is defined in the first side surface 37A of the connecting arm 37. In some embodiments, the first cable groove 34 is configured as an arc-shaped groove (with a central angle of at least 180 degrees) centered about the first pivot axis PA1. In a radial direction of the first cable groove 34, the third cable 61 and the fourth cable 62 extend along opposite sides of the first cable groove 34. The connecting part 31A is positioned adjacent to the first cable groove 34.

In the illustrated embodiments, the base assembly 40 (specifically, the base bottom plate 46) defines one or more through-holes through which the third cable 61 and the fourth cable 62 pass. Thus, the paths of the third cable 61 and the fourth cable 62 are determined by both the first cable groove 34 and the through-holes in the base bottom plate 46.

In some embodiments, as shown in FIG. 3 to FIG. 5, the execution assembly 10 further includes an insulating sleeve 12 and a sealing ring 13. The electrode component 11 is fixed to the insulating sleeve 12 (e.g., by means of adhesive or threaded connection). The second base 21 is also fixed to the insulating sleeve 12 (e.g., by means of adhesive or threaded connection), thereby connecting the second pivot assembly 20 to the execution assembly 10. The insulating sleeve 12 is configured to protects the second base 21 from arc damage during discharging of the electrode component 11, and thus it is made from a material with arc resistance properties. The sealing ring 13 is arranged at the connection between the electric wire 14 and the electrode component 11, functioning as waterproofing at the connection of the electric wire 14 to the electrode component 11. This prevents liquid from being infiltrating into the connection of the electric wire 14 to the electrode component 11 during the use or cleaning of the instrument, which could otherwise cause short circuits or incomplete cleaning, leading to instrument damage or potential injury to patients or operators. As illustrated in FIG. 5, an annular step surface 26A is provided on the inner wall of the wire through-hole 26 and configured to retain the sealing ring 13.

In some embodiments, the surgical instrument 100 further includes a shaft 50. The shaft 50 has a distal end where the end effector 90 is arranged, and has a proximal end connected to a driving device. The shaft 50 defines a hollow interior through which the first flexible transmission assembly 60 and the second flexible transmission assembly 70 pass to connect with the driving device, allowing the driving device to drive the first flexible transmission assembly 60 and the second flexible transmission assembly 70 to rotate the first pivot assembly 30 and the second pivot assembly 20.

In the present disclosure, the electric wire 14 is mechanically connected to the driving device at the rear end of the surgical instrument 100, enabling deployment and retraction of the electric wire 14.

In embodiments without the guiding pulley 87, the electric wire 14 needs to be released or retracted during pitch of the surgical instrument 100. Taking the orientation shown in FIG. 2 as an example, when the end effector 90 pitches outward from the plane of the paper, the driving device retracts the electric wire 14 to reduce the length of the electric wire 14 extending beyond the base bottom plate 46, preventing the electric wire 14 from detaching from the wire pulley 86. Conversely, when the end effector 90 pitches inward toward the plane of the paper, the electric wire 14 is released, preventing over stretch of the electric wire 14. To ensure coordinated retraction/release of the electric wire 14 with while pitch, the electric wire 14 may be driven simultaneously with the fourth cable 62. For example, both the electric wire 14 and the fourth cable 62 may be bound together within the shaft 50, allowing the fourth cable 62 to drive synchronous movement of the electric wire 14. Further, the wire pulley 86 is arranged adjacent to the first cable groove 34, facilitating synchronous retraction/release of the electric wire 14 14 and the fourth cable 62. It can be understood that in other embodiments, the electric wire 14 may be routed to the other side of the wire pulley 86 and driven synchronously with the third cable 61.

In the embodiments where the guiding pulley 87 is provided, the electric wire 14 needs to be released or retracted during yaw of the surgical instrument 100. Taking the orientation shown in FIG. 7 or FIG. 8 as an example, when the end effector 90 yaws counterclockwise, the driving device retracts the electric wire 14 to prevent accumulation within the wire passage 25. Such accumulation could cause excessive bending of the electric wire 14 and interference with the movement of the second pivot assembly 20. Conversely, when the end effector 90 yaws clockwise, the electric wire 14 is released, preventing over stretch of the electric wire 14. To ensure coordinated retraction/release of the electric wire 14 while pitch, the electric wire 14 may be driven synchronously with the first cable 71. For instance, the electric wire 14 and the first cable 71 may be bound together within the shaft 50, allowing the first cable 71 to drive synchronous movement of the electric wire 14. It can be understood that in other embodiments, the electric wire 14 may be routed to the other side of the guiding pulley 87 and driven synchronously with the second cable 62.

The base assembly 40 is arranged at the distal end of the shaft 50. The shaft 50 extends straight in the first direction D1, and the support body 47 is connected to the shaft 50. In some embodiments, the support body 47 is adhesively bonded to the shaft 50, which also ensures the airtightness of the surgical instrument 100.

In the illustrated embodiments, the shaft 50 includes a support tube 51 and an insulating sleeve 52. The support tube 51 is connected with the base assembly 40. The insulating sleeve 52 is fitted over or coated on the outer circumference of the support tube 51. In some embodiments, the support tube 51 is made of a high-strength rigid material for supporting. The insulating sleeve 52 is made of an insulating material, which prevents direct contact between the support tube 51 and human tissue, thereby avoiding electrical shock hazards.

In some other embodiments, the shaft 50 may be integrally made of a rigid insulating material, such as fiberglass or plastic.

In some embodiments, a sealing component is provided between the base assembly 40 and the shaft 50. For instance, the surgical instrument 100 further includes a sealing gasket 85 arranged inside the support body 47 and connected (e.g., adhesively bonded) to the base bottom plate 46. The sealing gasket 85 defines one or more through-holes corresponding to the through-holes in the base bottom plate 46, allowing the drive cable of the first flexible transmission assembly 60, the drive cable of the second flexible transmission assembly 70, and the electric wire 14 to pass through. In some embodiments, the through-holes in the sealing gasket 85 are featured with controlled interference fits against both the drive cables and the electric wire, ensuring sealing performance and prevent fluids from entering the instrument tube from the distal end.

According to the first aspect of the embodiments of the present disclosure, the surgical instrument 100 achieves miniaturization, particularly reducing its radial dimension down to 5 mm or even 4 mm, through optimized routing configurations of the electric wire 14, the first flexible transmission assembly, and the second flexible transmission assembly. This advancement in surgical instruments maintains structural integrity of the electric wire and all other components while ensuring operational reliability and service life.

According to the second aspect, the present disclosure provides a surgical robot. In some embodiments, the surgical robot includes a robotic arm and the aforementioned surgical instrument 100. The surgical instrument 100 is operably connected to the robotic arm. The robotic arm features multiple degrees of freedom to facilitate flexible manipulation of the surgical instrument 100 during surgical procedures. Further, the robotic arm is provided with a driving device (e.g., a motor), and the transmission mechanism of the surgical instrument 100 is mechanically connected to the driving device. This configuration enables the driving device to actuate the end effector 90 for yaw and/or pitch.

The surgical robot according to the present disclosure includes all the features and effects of the surgical instrument described herein.

In interpreting the scope of the present application, the term "comprising" and its derivatives as used herein are intended to be open-ended terms, designating the presence of the stated features, elements, components, groups, assemblies, and/or steps described herein, but not excluding the presence of other features, elements, components, groups, assemblies, and/or steps not described herein. This concept also applies to words with similar meanings, such as the terms "including", "having", and their derivatives.

The terms "attached" or "attach" used herein include: a configuration in which a component is directly fixed to another component by directly fixing the component to the other component; a configuration in which a component is indirectly fixed to another component by fixing the component to an intermediate component, which in turn is fixed to the other component; and a configuration in which one component is integral with another component, i.e., one component is essentially part of the other component. The definition also applies to terms with similar meanings, such as "connect", "link", "couple", "install", "bond", "fix", and their derivatives. Moreover, the degree terms used here, such as "basically", "approximately", and "substantially", indicate the degree of deviation that modifies the terms without significantly altering the final result.

Unless otherwise defined, the technical and scientific terms used herein have the same meanings as those commonly understood by those skilled in the art. The terms used herein are only for the purpose of describing the specific implementation and are not intended to limit the present disclosure. The features described in one embodiment may be applied separately or in combination with other features in another embodiment, unless such features are not applicable in that other embodiment or otherwise specified.

The present disclosure has been described through the aforementioned embodiments. However, it should be noted that the aforementioned embodiments are provided for illustrative purpose only and are not intended to limit the present disclosure to the scope of the described embodiments. Furthermore, those skilled in the art will understand that the present disclosure is not limited to the aforementioned implementations, and that various modifications and variations may be made in accordance with the teachings of the present disclosure, all of which fall within the scope of the present disclosure as claimed. According to the teachings of the present disclosure, more variations and modifications can be made, and all these variations and modifications fall within the scope of protection claimed by the present disclosure.

## Claims

1. A surgical instrument comprising an end effector, the end effector comprising:
a base assembly;
a first pivot assembly pivotably connected to the base assembly about a first pivot axis;
a first flexible transmission assembly connected to the first pivot assembly, the first flexible transmission assembly being configured to drive the first pivot assembly to pivot relative to the base assembly about the first pivot axis;
a second pivot assembly pivotably connected to the first pivot assembly about a second pivot axis that is non-parallel to the first pivot axis;
a second flexible transmission assembly connected to the second pivot assembly, the second flexible transmission assembly being configured to drive the second pivot assembly to pivot relative to the first pivot assembly about the second pivot axis; and
an execution assembly connected to the second pivot assembly, the execution assembly comprising an electrode component and an electric wire for powering the electrode component, and the electric wire passing through the second pivot assembly, the first pivot assembly, and the base assembly;
wherein the second pivot assembly has an end away from the electrode component and defining a wire passage, the electric wire is movably received in the wire passage, the wire passage extends toward the electrode component, the wire passage is defined at least by two first wall surfaces facing to each other and a second wall surface transverse to the two first wall surfaces, and the second pivot axis perpendicularly passes through the two first wall surfaces.

2. The surgical instrument of claim 1, wherein the second wall surface and the electrode component are located on a same side of the second pivot axis.

3. The surgical instrument of claim 1 or 2, wherein the second pivot assembly has an end adjacent to the electrode component and defining a wire through-hole communicated with the wire passage, and the electric wire passes through the wire passage and enters into the wire through-hole; and
the second wall surface features a third curved surface and transitions to an inner wall of the wire through-hole via the third curved surface, the third curved surface features a third arc parallel to the third wall surface, and the third curved surface is configured to guide the electric wire in rotation of the second pivot assembly relative to the first pivot assembly.

4. The surgical instrument of claim 3, wherein the third curved surface is tangential to the inner wall of the wire through-hole.

5. The surgical instrument of claim 3 or 4, wherein the third curved surface has an end extending away from the wire through-hole to an opening of the wire passage.

6. The surgical instrument of any one of claims 1 to 5, wherein the second pivot assembly has an end face facing away from the electrode component and featuring a second curved surface axially centered on the second pivot axis, the second curved surface features a second arc parallel to the first wall surface, and the wire passage has an opening extending along the second arc at least to two ends of the second arc.

7. The surgical instrument of claim 6, wherein a central angle of the second arc is greater than or equal to 180 degrees.

8. The surgical instrument of claim 6 or 7, wherein the opening of the wire passage extends along the second arc beyond two ends of the second arc.

9. The surgical instrument of any one of claims 1 to 8, wherein a distance between the two first wall surfaces is matched to a diameter of the electric wire.

10. The surgical instrument of any one of claims 1 to 9, wherein the first pivot axis is perpendicular to the second pivot axis.

11. The surgical instrument of any one of claims 1 to 10, wherein the second flexible transmission assembly comprises a first cable and a second cable for respectively driving the second pivot assembly to rotate in opposite directions about the second pivot axis.

12. The surgical instrument of claim 11, wherein an extension trajectory of the first cable in the second pivot assembly and an extension trajectory of the second cable in the second pivot assembly lie in a first plane, the first plane is perpendicular to the second pivot axis, and the first plane lies outside the wire passage.

13. The surgical instrument of claim 11, wherein an extension trajectory of the first cable in the second pivot assembly lies in a first plane, an extension trajectory of the second cable in the second pivot assembly lies in a second plane, the first plane and the second plane are parallel to each other and both perpendicular to the second pivot axis, and the wire passage is located between the first plane and the second plane.

14. The surgical instrument of any one of claims 11 to 13, further comprising:
a first pulley pivotably connected to the base assembly about the first pivot axis; and
a second pulley pivotably connected to the base assembly about the first pivot axis, the second pulley being spaced apart from the first pulley along the first pivot axis;
wherein the first pulley is configured to guide the first cable, the second pulley is configured to guide the second cable, and a winding direction of the first cable on the first pulley is opposite to a winding direction of the second cable on the second pulley.

15. The surgical instrument of claim 14, wherein a longitudinal axis of the base assembly extends in a first direction, the first pivot axis is perpendicular to the first direction, and the second pivot axis is perpendicular to the first direction when the first pivot assembly is in a neutral position relative to the base assembly.

16. The surgical instrument of claim 15, further comprising:
a third pulley pivotably connected to the base assembly about a third pivot axis;
wherein the third pivot axis is parallel to the first pivot axis, the first pivot axis and the third pivot axis define a third plane, the first cable rides on the first pulley on one side of the third plane, and the first cable rides on the third pulley on the other side of the third plane.

17. The surgical instrument of claim 16, wherein the first cable extends from the second pivot assembly in the first direction to the first pulley when the first pivot assembly is in a neutral position relative to the base assembly, and further extends from the third pulley in the first direction to the base assembly.

18. The surgical instrument of any one of claims 15 to 17, further comprising:
a fourth pulley connected to the first pivot assembly, an axis of the fourth pulley being parallel to the first pivot axis, and the first pivot axis and the axis of the fourth pulley defining a fourth plane;
wherein the second cable rides on the fourth pulley on one side of the fourth plane, and the second cable rides on the second pulley on the other side of the fourth plane.

19. The surgical instrument of claim 18, wherein the second cable extends from the second pivot assembly in the first direction to the fourth pulley when the first pivot assembly is in a neutral position relative to the base assembly, and further extends from the second pulley in the first direction to the base assembly.

20. The surgical instrument of claim 18 or 19, wherein the first pivot assembly comprises a first base, and the first base comprises:
a base plate extending parallel to the first pivot axis and the second pivot axis, the base plate defines one or more through-holes through witch the first cable and the electric wire pass;
a support arm connected to a side of the base plate facing the second pivot assembly and extending perpendicularly to the base plate, the support arm being configured to support and connect the second pivot assembly; and
a connecting arm connected to a side of the base plate facing the base assembly and extending perpendicularly to the base plate, the connecting arm being configured to be pivotably connected to the base assembly.

21. The surgical instrument of claim 20, wherein the connecting arm comprises a first side surface and a second side surface that are transverse to the first pivot axis and face away from each other;
wherein the first flexible transmission assembly is connected to the first side surface of the connecting arm, and the electric wire is located at a side the first side surface oriented after the electric wire passing through the base plate.

22. The surgical instrument of claim 21, wherein the first pulley is arranged at the side the first side surface oriented, and the second pulley and the fourth pulley are arranged at a side the second side surface oriented.

23. The surgical instrument of claim 22, further comprising:
a wire pulley rotatably connected to the base assembly about the first pivot axis and located between the first pulley and the connecting arm;
wherein the wire pulley is configured to guide the electric wire.

24. The surgical instrument of any one of claims 1 to 23, wherein the first flexible transmission assembly comprises a third cable and a fourth cable for respectively driving the first pivot assembly to rotate in opposite directions about the first pivot axis.

25. The surgical instrument of claim 24, wherein the electric wire is movable synchronously with the third cable or the fourth cable.

26. The surgical instrument of any one of claims 1 to 24, wherein a guiding surface is provided in the wire passage, the guiding surface extends about the second pivot axis, and the electric wire rides on the guiding surface when the second pivot assembly is in a neutral position relative to the first pivot assembly.

27. The surgical instrument of claim 26, wherein the second pivot assembly further comprises a guiding pulley rotatably mounted in the wire passage about the second pivot axis, and the guiding surface comprises an outer peripheral surface of the guiding pulley.

28. The surgical instrument of claim 26 or 27, wherein the second flexible transmission assembly comprises a first cable and a second cable for respectively driving the second pivot assembly to rotate in opposite directions about the second pivot axis, and the electric wire is movable synchronously with the first cable or the second cable.

29. The surgical instrument of any one of claims 1 to 28, wherein a sealing component is provided at a junction where the electric wire is connected to the electrode component.

30. The surgical instrument of any one of claims 1 to 29, further comprising:
a shaft;
wherein the end effector is arranged at a distal end of the shaft, and a sealing component is provided between the end effector and the shaft.

31. The surgical instrument of claim 30, wherein the shaft comprises:
a support tube, configured to be connected to the base assembly; and
an insulating sleeve made of an insulating material, the insulating sleeve being fitted over an outer periphery of the support tube or being coated on an outer periphery of the support tube.

32. A surgical robot, comprising:
a robotic arm; and
the surgical instrument of any one of claims 1 to 31, the surgical instrument being operably connected to the robotic arm.
